# EUROPEAN PATENT APPLICATION

(11) **EP 2 810 572 A1**
(43) Date of publication of application: **10.12.2014**
(21) Application number: 14171552.4
(22) Date of filing: 06.06.2014
(51) Int. Cl.: A41C 3/04

(54) **Pumping/nursing bra**

(30) Priority: 07.06.2013 US 201361832592 P; 04.02.2014 US 201414172812
(71) Applicant: Simple Wishes LLC, Sacramento, CA 95860 (US)
(72) Inventor: ABBASZADEH, Debra, Sacramento, CA California 95826 (US)
(74) Representative: Boult Wade Tennant

(57) **Abstract**

A nursing and pumping bra (11), comprising: an inner pumping panel (19) comprising at least one covering for a wearer's breast, the at least one covering comprising a first layer of fabric (54) that overlaps a second layer of fabric (56) to cover an opening, the at least one covering having stitching extending across a portion of the first layer of fabric and a portion of the second layer of fabric, and the at least one covering is operative to provide support during milk expression for at least one of the wearer's breasts and a portion of a breast pump body inserted through the opening; and an exterior front surface (17) comprising a first panel of fabric (16) that overlaps at least a portion of a second panel of fabric (18), the first and the second panels operative to be moved to expose the at least one covering of the inner pumping panel.

## Description

### FIELD OF THE INVENTION

The present invention is directed to a device to assist a woman in pumping breast milk.

### BACKGROUND

When using a breast pump, the mother must manually hold the breast flange or shield over the exposed portion of the breast. Due to the length of time required to express milk when using a pump, a woman will often express milk from both breasts simultaneously. To express milk from both breasts, the woman must hold the breast flange or shield with each hand against her breast, leaving her in an awkward position, making it difficult to do something as simple as operating the breast pump. As a result, the nursing mother will not have the full use of both hands to perform other tasks. It would be advantageous to develop a device that would enable a mother to use a breast pump while allowing her the free use of her hands to perform other tasks.

Hands-free pumping affords a mother the ability to simultaneously massage her breasts to enhance milk let down, a procedure that is not possible with most breast pumps currently in use, or to simply engage in other pursuits when the milk is being pumped. None of prior art addresses the combination of features disclosed in the present invention.

### SUMMARY

The present invention is directed to a pumping/nursing bra that assists in creating a tight seal between the woman's breasts and a breast shield of a breast pump. The front of the pumping/ nursing bra is provided with two pairs of openings, each of the openings in a covering one of the woman's breasts. Each of the pair of openings is covered by two overlapping layers of fabric included in a pumping panel. The pumping/nursing bra is provided with top and bottom elastic edges to prevent the bra from slipping down, as well as supporting the woman's breasts. An adjustable shoulder strap is attached to both sides of the pumping/nursing bra. One embodiment of the present invention includes elastic loops provided on either side of the bra attached to the shoulder straps designed to hook around the breast shield to aid in the support of the breast shield while pumping. Embodiments of the present invention include providing a neck strap extending around the back of the woman's neck, the neck strap attached to the top line of the pumping/nursing bra. The neck strap includes either a single hook attached to the pumping/ nursing bra or a multitude of hooks attached to the pumping/nursing bra. The top line of the pumping/nursing bra would include one or more loops attaching the strap to the pumping/nursing bra. The purpose of the neck strap is to ensure that the pumping/nursing bra remains in place during breast pumping, particularly when the breast pump bottle becomes full with milk.

It is, therefore, an object of the present invention to provide a pumping/nursing bra containing one or more elastic loops used to hook around the breast shield.

Yet another object of the present invention is to provide a pumping/ nursing bra provided with a strap encircling the neck of the woman having at least one hook attached to the top line of the pumping/ nursing bra to ensure that the pumping/nursing bra remains in place during the use of a breast pump on one or more of the woman's breasts.

A further object of the present invention is to provide a pumping/nursing bra including a top line attached to the bra allowing a strap to be attached thereto.

Other objects and advantages of the present invention will become apparent from the following detailed description when viewed in conjunction with the accompanying drawings, which set forth certain embodiments of the invention.

### BRIEF DESCRIPTION OF THE DRAWINGS

Figures 1A, 1B and 1C are views of one embodiment of the pumping/nursing bra.
Figures 2A, 2B, and 2C are views of a second embodiment of the pumping/ nursing bra.
Figures 3A, 3B and 3C are views of a third embodiment of the present invention.
Figure 4 is a front view of a variation of the pumping/nursing bra shown in Figures 3A, 3B and 3C.

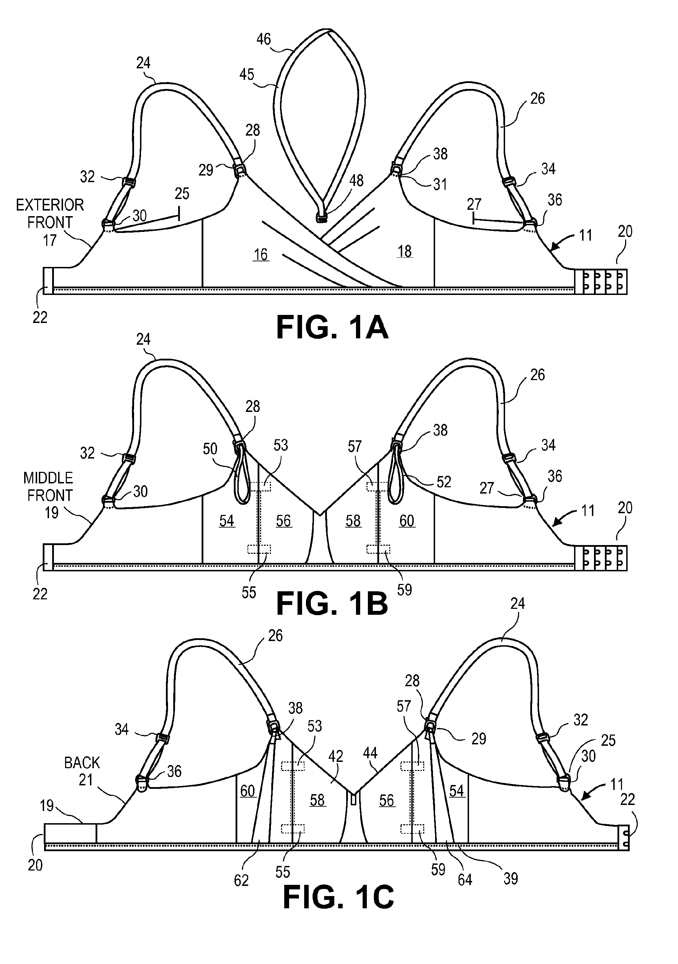

Figures 1, 2 and 3 having a neck strap attached to the top line of the bra.

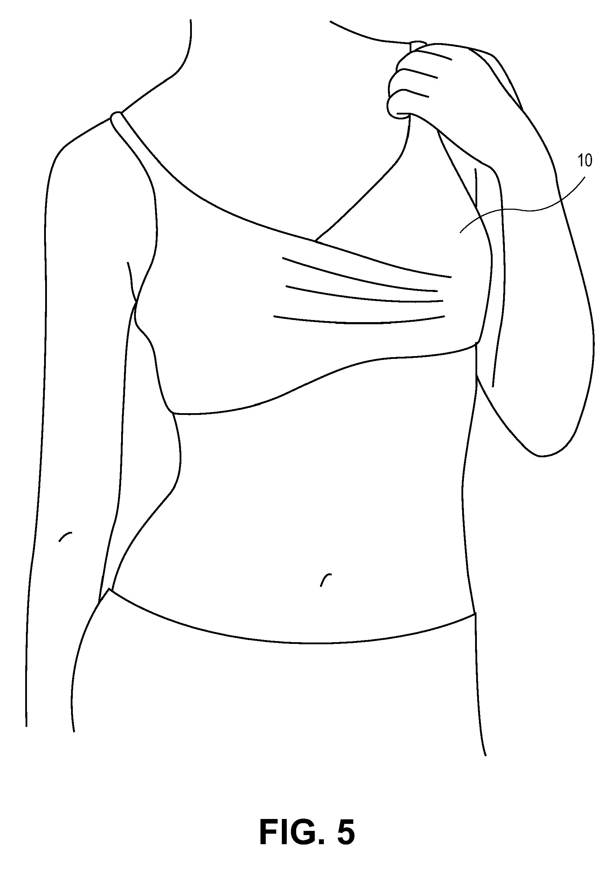

Figure 5 is a view showing the bra being worn without the neck strap.

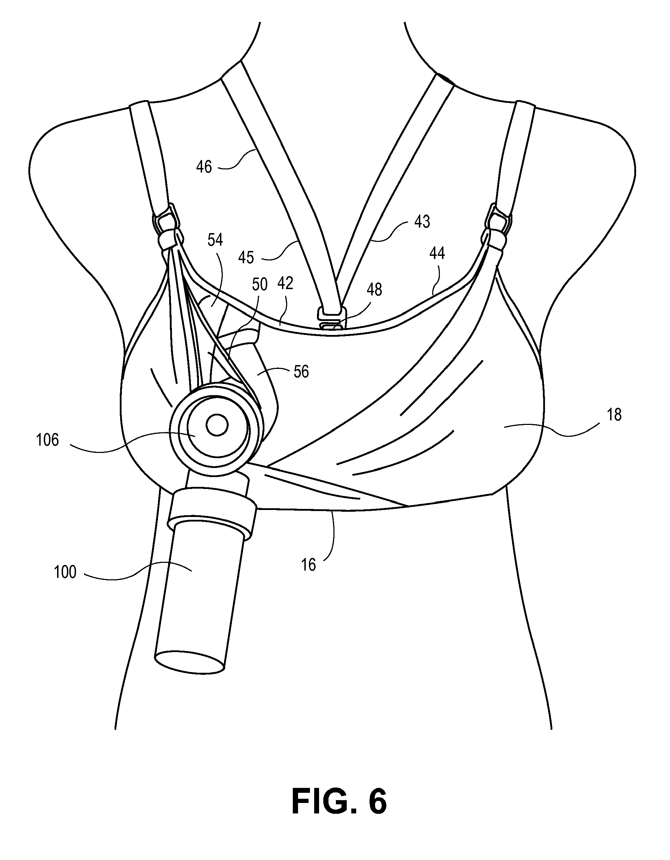

Figure 6 is a view showing the bra being worn with the neck strap attached to the top line of the bra and shows an attached single pumping unit.

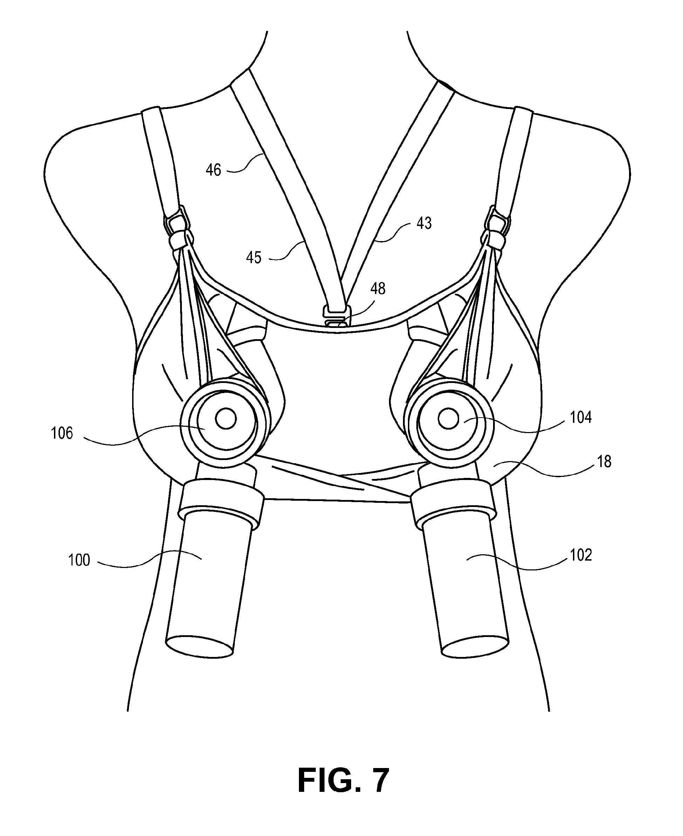

Figure 7 is a view of the pumping/nursing bra having the neck strap attached to the top line of the bra and shows two attached pumping units.

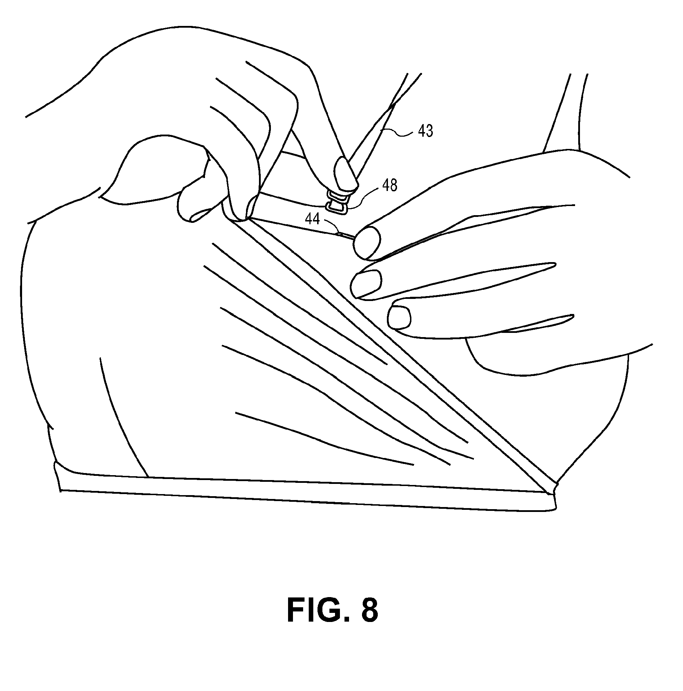

Figure 8 is a view of the pumping/nursing bra illustrating the hook attached to the strap.

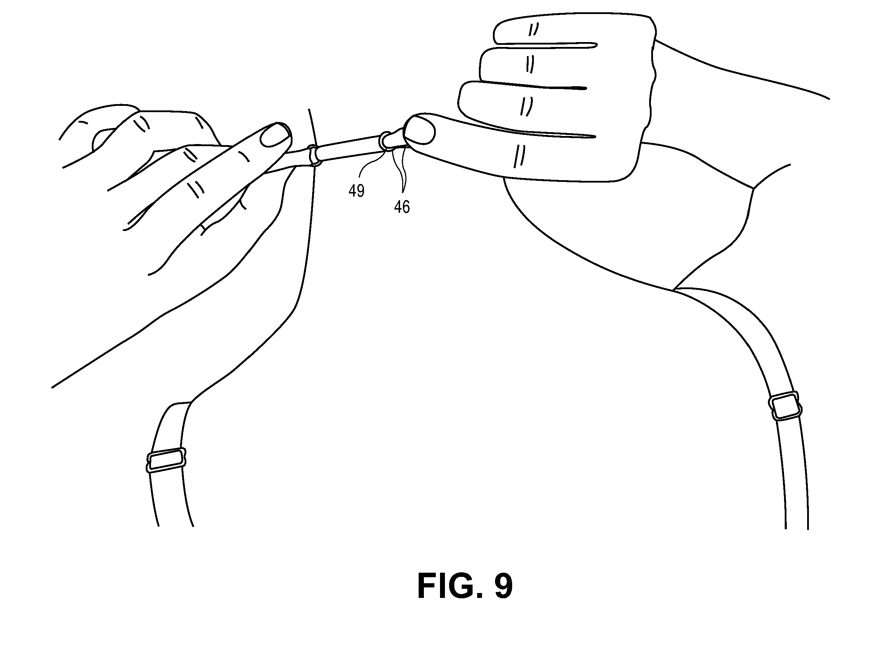

Figure 9 is a view of the strap extending around a woman's neck.

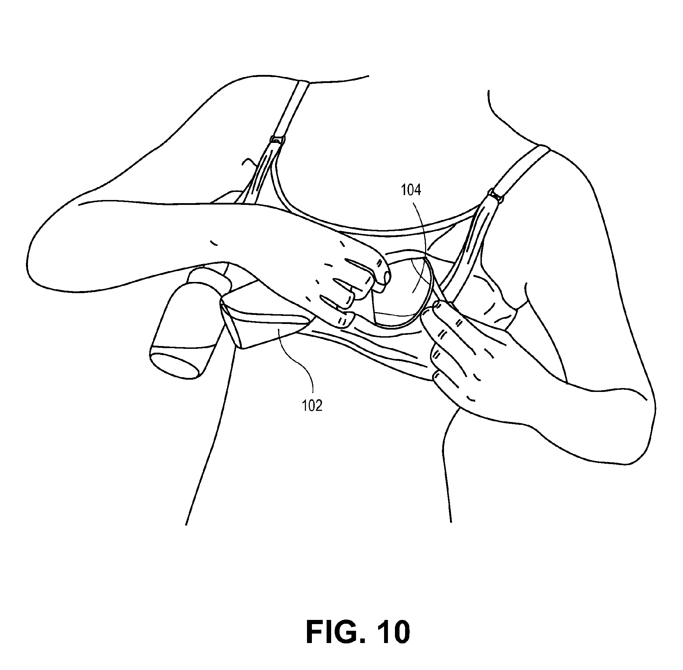

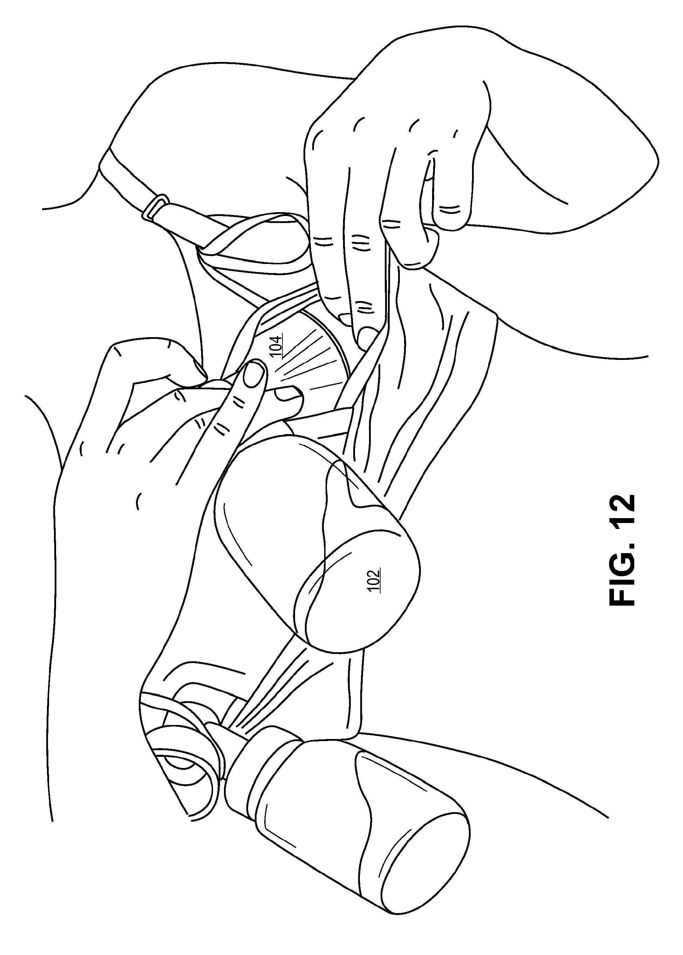

Figures 10, 11 and 12 are views showing a woman inserting a breast shield between overlapping layers of a pumping panel.

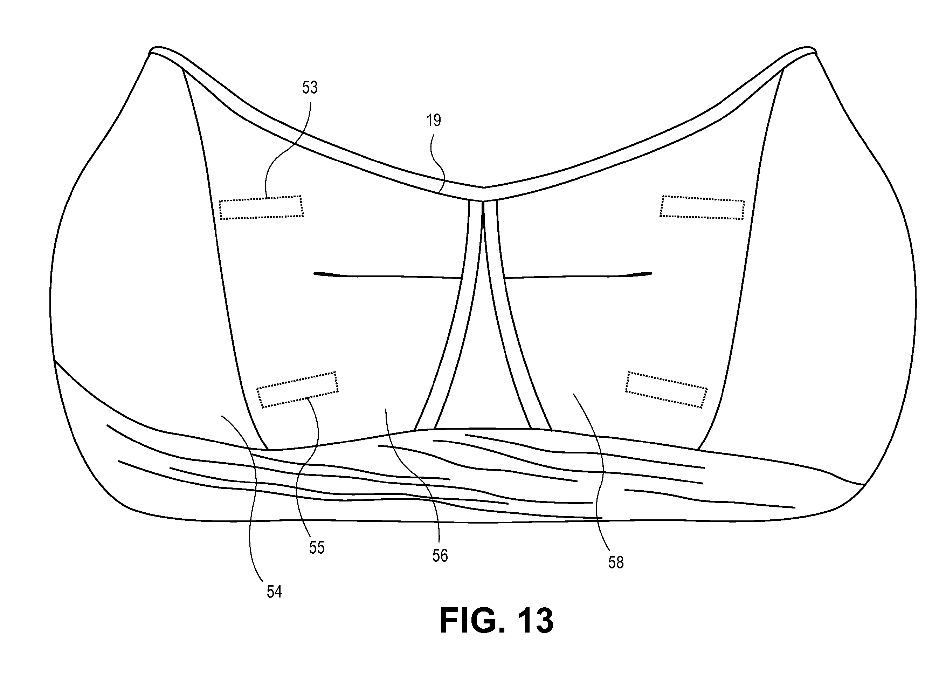

Figure 13 is a view of the overlapping layers of the pumping panel.

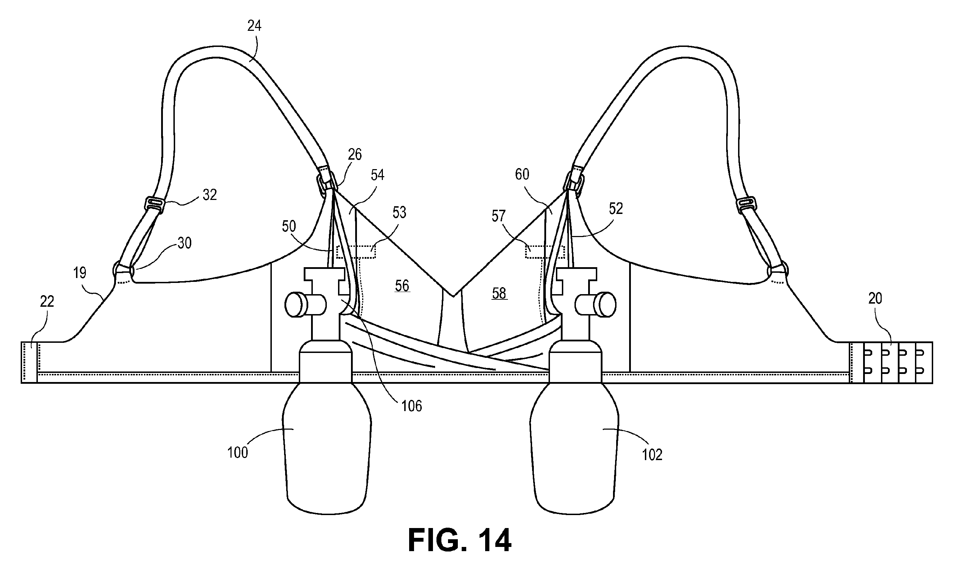

Figure 14 is a front view of the pumping panel of the pumping/nursing bra showing the attachment of two breast pumps supported by loops.

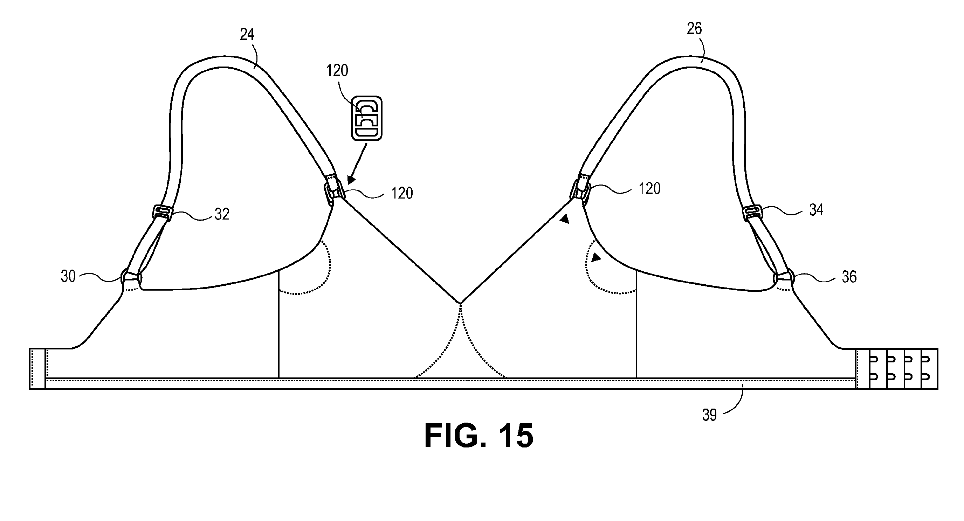

Figure 15 is a front view of a variation of the pumping panel showing the use of a double hook attachment device.

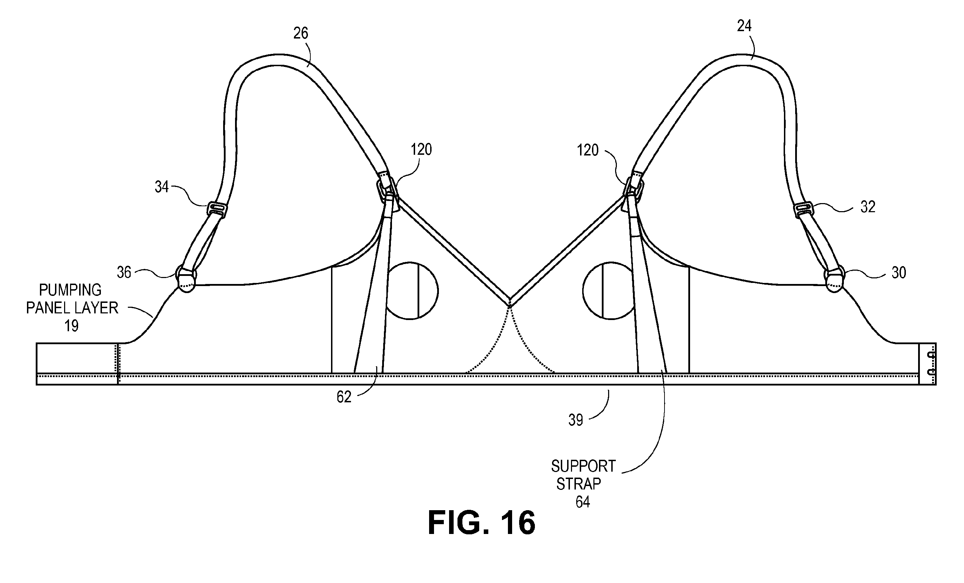

Figure 16 is a rear view of the pumping panel shown in Figure 15.

### DESCRIPTION OF THE DRAWINGS

The detailed embodiments of the present invention are disclosed herein. It should be understood, however, that the disclosed embodiments are merely exemplary of the invention, which may be embodied in various forms. Therefore, the details disclosed herein are not to be interpreted as limiting, but merely as a basis for teaching one skilled in the art how to make and/or use the invention. The present invention incorporates by reference the entire teachings of U.S. Patent No. 8,192,247 and 8,323,070 owned by the same inventor of this application.

Figures 1A, 1B and 1C illustrate a first embodiment of the present invention. Figure 1A illustrates a pumping/nursing bra 11 is provided with an exterior front surface 17. Figure 1B shows the front surface of a pumping panel19 not covered by the exterior front surface 17. Figure 1C illustrates the rear surface 21 of the pumping panel 19. The exterior front surface 17 includes a first panel 16 crossing in front of a second panel 18, with both of the panels 16, 18 providing a smooth cover over the pumping panel 19. The pumping panel 19 includes a first pair of overlapping layers 54, 56 and a second pair of overlapping layers 58, 60. During pumping, overlapping layers 54 and 56 are separated to uncover one of the woman's breast. Overlapping layers 58 and 60 are separated to uncover the other breast of the women. The panels 16, 18 help to prevent the breast pumps and breast shields from slipping downward from the woman's breast. A first shoulder strap 24 is provided at a first end 29 of the pumping/ nursing bra with a hook 28 having a first end attached to one end of a fabric stay 64. A second end 25 of the first shoulder strap 24 is attached to the panel 16 by a hook 30. The second end of the fabric stay 64 extends to, and is attached to the bottom edge 39 of the pumping panel 19. A second end of the hook 28 is attached to one end of the pumping panel 19 and one end of the panel 16 preferably by a sewn connection. Both the first and second ends of the hook 28 are designed to be attached to one another, connecting the first panel16 to the pumping panel 19. A second shoulder strap 26 contains a hook 38 having a first end 31 attached to one end of a fabric stay 62. The second end of the fabric stay 62 extends to, and is attached to the bottom edge 39. A second end of the hook 38 is attached to one end of the pumping panel 19 and one end of the panel 18. Preferably both the first and second ends of the hook 38 are designed to be attached to one another, connecting the second panel 18 to the pumping panel 19. The second shoulder strap 26 contains a hook member 36 connected to the pumping/nursing bra. A slide member 32 provided on the first shoulder strap 24 and a slide member 34 are provided on the second shoulder strap 26 is used to adjust the length of each of the shoulder straps 24, 26. Adjustable hook and eye closures 20, 22 are used to secure the pumping/nursing bra around the chest of a woman.

Figures 15 and 16 show the use of double hooks 120 to be used instead of the hooks 28, 38. The first end of the double hook is sewn on the shoulder strap and the second end of the double hook is sewn to the fabric stay 62, 64. The outer or exterior layer 17 hooks to the top catch of the double hook 120 and the pump support hooks to the bottom catch of the double hook. The extension layer 17 and pump support panel can be hooked and unhooked from the catches.

The fabric stay 62 extends from the double hook 120 to the bottom 39 of the pumping panel 19. Similarly, the fabric stay 64 extends from the double hook 120 to the bottom surface 39 of the pumping panel 19. These exterior layer 17 and pump support layer 19 can be unhooked when a mother is breast feeding and re-attaches when the woman is not breast feeding. The fabric stays 62, 64 keep the shoulder strap secure when the panels 16 and 18 are moved to allow for breast feeding. The pumping panel 19 is provided with top rectangular shaped stitching panel 53 and a bottom rectangular shaped stitching panel 55 extending across a portion of the overlapping sections 54, 56 of the pumping panel 19. Similarly, a top rectangular stitching panel 57 and a bottom rectangular stitching panel 59 extend across a portion of the overlapping sections 58, 60 of the pump support panel19. As shown in Figure 1B, a first elastic loop 50 is attached to the first shoulder strap 24 or is sewn into the support panel 19. Additionally, a second elastic loop 52 is attached to the second shoulder strap 26 or is sewn into the support panel 19. The purpose of elastic loops 50, 52 is to hook around the breast shield when in use, to support the breast shield, particularly when the breast pump is full of milk, as shown in Figure 14.

The embodiment shown in Figure 1A includes a strap 46 having a single loop 45 joined at its bottom by a hook member 48 encircles the neck and then extends downwardly to the pumping/ nursing bra 11. The hook member 48 is attached to an opening provided in a top line 42, 44 at approximately the center of the pumping panel 19. The purpose of the neck strap is to maintain the pumping/nursing bra 11 in place while the woman is pumping breast milk as well as to add stability to the pumping/nursing bra 11, particularly when the breast pump is full of milk.

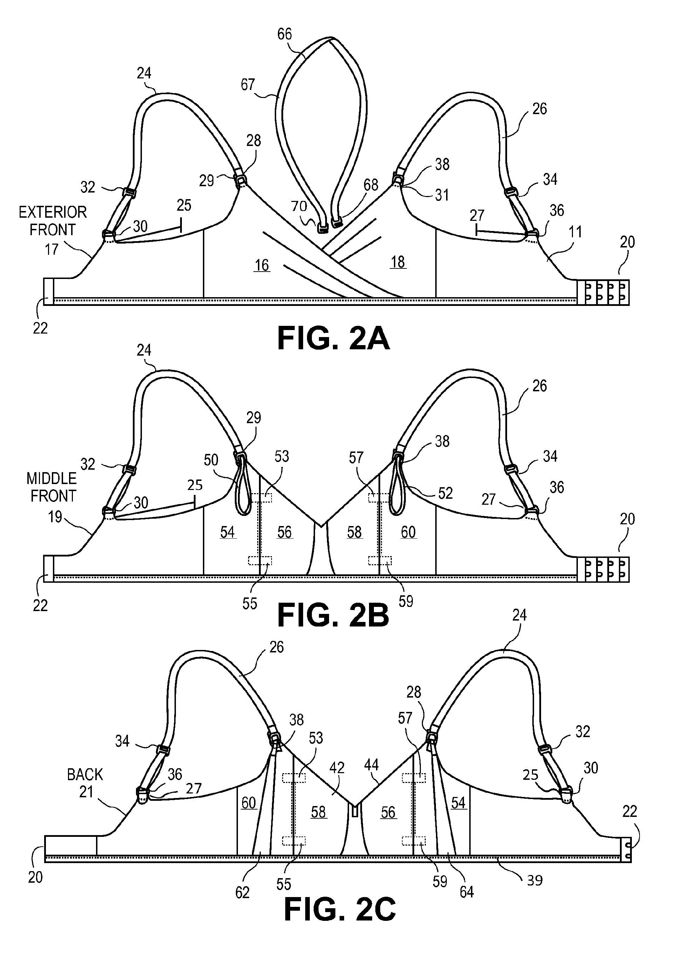

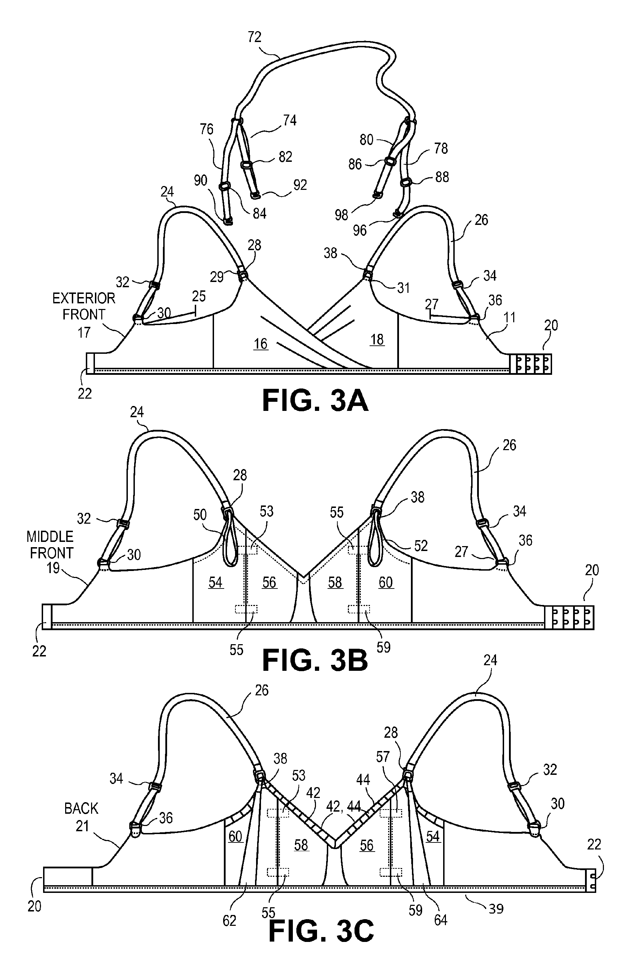

The embodiment shown in Figures 2A, 2B and 2C is similar to the embodiment shown in Figures 1A, 1B and 1C, with the exception of the use of strap 66 in place of the neck strap 46. Neck strap 66 encircles the neck of the women and includes a single loop 67 provided with hooks 68, 70 at its lower end. Each of the hooks 68, 70 attach to the top line 42, 44 close to the center of the pumping panel 19. The embodiment shown in Figures 3A, 3B and 3C employs a neck strap 72 in lieu of the straps 46, 66 illustrated in the first two embodiments of the present invention. The strap 72 contains extension sections 74, 76, 78, 80, each of which is provided with a hook 92, 90, 96, 98 respectively. Each of the hooks 90, 92, 96, 98 is attached to the top line 42, 44. As can be appreciated, the greater the number of extensions from the strap 72 and consequently the number of hooks extending from each of the extensions the stability of the pumping/ nursing bra during pumping increases.

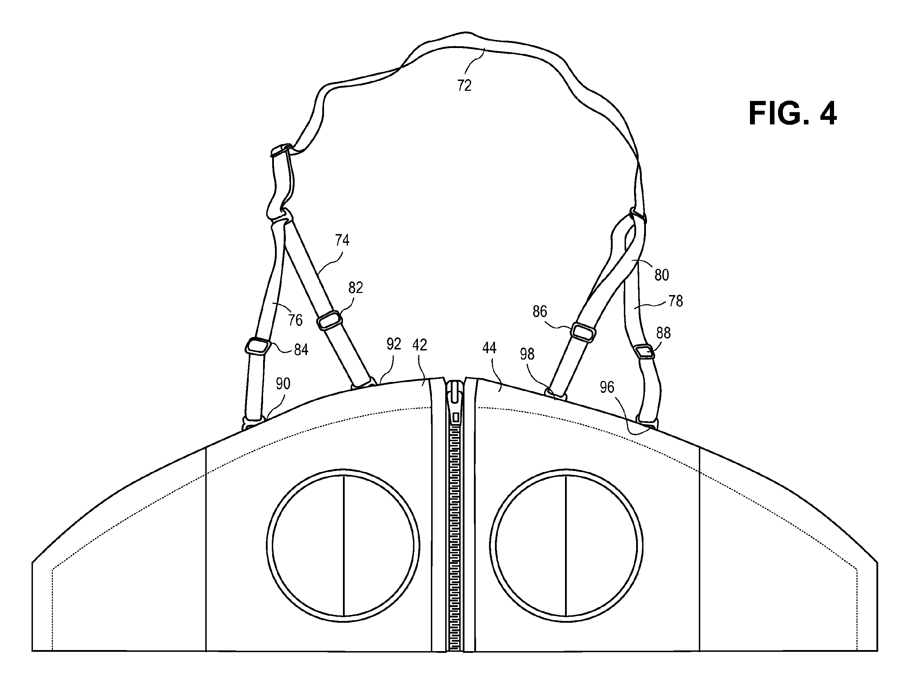

Figure 4 shows a variation of the embodiment shown in Figures 3A, 3B and 3C, in which the strap 72 is attached to the top line 42, 44 of the pumping/nursing bra. Adjustable slides 82, 84, 86, 88 would adjust the length of the strap 72 to accommodate different size women.

Figures 5-13 illustrate the manner in which the pumping/ nursing bra is to be utilized. The pumping/nursing bra 10 is designed to worn for the entire day as shown in Figure 5. When breast milk pumping is to be initiated, one of the straps 46, 66, 72 would encircle the woman's neck and be attached to the top layer of the pumping panel. As shown in Figure 6, the woman moves the panel 16 slightly downward, allowing the woman to provide an opening between panels 54, 56 to insert a breast shield between the panels 54, 56 to connect the woman's breast to a pumping bottle 100 through a breast shield 106.

Figure 7 illustrates the use of two pumping bottles 100, 102 connected to breast shields 106, 104 respectively.

Figure 8 shows the manner in which the hook 48 of the strap 46 is attached to a loop provided within the top layer 44.

Figure 9 shows the manner in which the strap 46 encircles the neck of the woman. A slide 49 is provided to adjust the length of the strap 46.

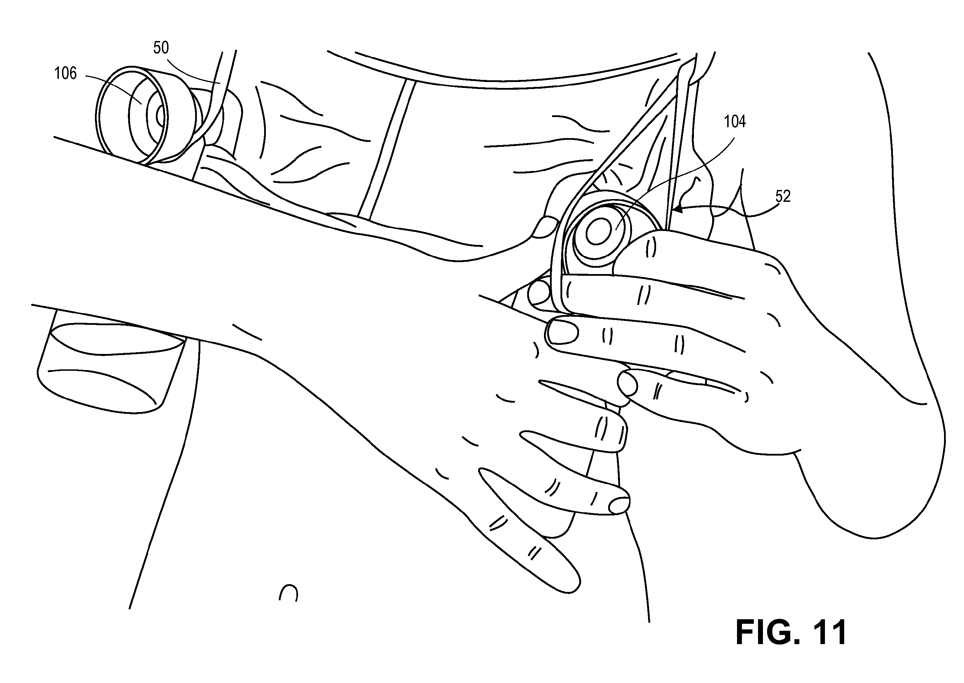

Figures 10, 11 and 12 show the manner in which the breast shield is inserted through the pumping/nursing bra 10. As illustrated in Figure 11 and Figure 14, elastic loop 52 is provided around the breast shield 104 and elastic loop 50 is provided around breast shield 106.

Figure 13 illustrates the pumping panel 19, as well as the rectangular shaped stitching 53, 55 provided across a portion of the overlapping panels 54, 56.

It is noted that based upon the comfort of the woman during breast milk pumping, the woman may choose to utilize the elastic loops 50, 52 to hook around the breast shield, without the utilization of any of the straps 46, 66, 72. Alternatively, the woman might utilize straps 46, 66, or 72 without employing the elastic loops 50, 52. Furthermore, the woman might utilize both the elastic loops 50, 52 along with one of the straps 46, 66, 72.

While the preferred embodiments have been shown and described, it will be understood that there is no intent to limit the invention by such disclosure, but rather, is intended to cover all modifications and alternate constructions falling within the spirit and scope of the invention.

Figure 13 illustrates the pumping panel 19, as well as the rectangular shaped stitching 53, 55 provided across a portion of the overlapping panels 54, 56.

It is noted that based upon the comfort of the woman during breast milk pumping, the woman may choose to utilize the elastic loops 50, 52 to hook around the breast shield, without the utilization of any of the straps 46, 66, 72. Alternatively, the woman might utilize straps 46, 66, or 72 without employing the elastic loops 50, 52. Furthermore, the woman might utilize both the elastic loops 50, 52 along with one of the straps 46, 66, 72.

While the preferred embodiments have been shown and described, it will be understood that there is no intent to limit the invention by such disclosure, but rather, is intended to cover all modifications and alternate constructions falling within the spirit and scope of the invention.

The present application discloses subject matter in correspondence with the following numbered clauses:
Clause 1. A nursing and pumping bra, comprising: an inner pumping panel comprising at least one covering for a wearer's breast, the at least one covering comprising a first layer of fabric that overlaps a second layer of fabric to cover an opening, the at least one covering having stitching extending across a portion of the first layer of fabric and a portion of the second layer of fabric, and the at least one covering is operative to provide support during milk expression for at least one of the wearer's breast and a portion of a breast pump body inserted through the opening; and an exterior front surface comprising a first panel of fabric that overlaps at least a portion of a second of panel of fabric, the first and the second panels operative to be moved to expose the at least one covering of the inner pumping panel.
Clause 2. The nursing and pumping bra of clause 1, wherein the breast pump body sits within the opening and is supported by the first and the second layers of fabric.
Clause 3. The nursing and pumping bra of clause 1, wherein the first and the second panels provide support for the breast pump body when the inner pumping panel is not covered.
Clause 4. The nursing and pumping bra of clause 1, wherein the inner pumping panel comprises two coverings, wherein a second covering comprises a third layer of fabric and a fourth layer of fabric to cover an opening, the second covering having stitching extending across a portion of the first layer of fabric and a portion of the second layer of fabric.
Clause 5. The nursing and pumping bra of clause 1, further comprising at least one shoulder strap, a fabric stay, and a strap selectively attached to the nursing and pumping bra to fit around the wearer's neck and provide support for the portion of the breast pump body.
Clause 6. The nursing and pumping bra of clause 1, further comprising at least one elastic loop attached to the nursing and pumping bra to fit around a breast shield and secure the breast shield in place within the opening.
Clause 7. The nursing and pumping bra of clause 1, wherein the portion of the breast pump body is a breast shield.
Clause 8. The nursing and pumping bra of clause 1, wherein the first panel crosses over the second panels in a closed state to at least partially cover the inner pumping panel
Clause 9. A nursing and pumping bra comprising: a pumping panel of at least one layer of fabric, the pumping panel having at least one opening and fits around a chest of a wearer; and at least one loop of a fabric attached to the pumping panel, the at least one loop operative to support at least a portion of a breast pump body sitting within the opening of the pumping panel.
Clause 10. The nursing and pumping bra of clause 9, wherein the at least one loop is an elastic loop sewn into the pumping panel.
Clause 11. The nursing and pumping bra of clause 9, wherein the at least one loop is an elastic loop sewn into a shoulder strap on the pumping panel.
Clause 12. The nursing and pumping bra of clause 9, wherein the at least one loop fits around a breast shield of the breast pump body and secures the breast pump body.
Clause 13. The nursing and pumping bra of clause 9, wherein the pumping panel is an inner panel that is covered by an exterior set of panels.
Clause 14. A nursing and pumping bra comprising: at least one panel of fabric to fit around a wearer's chest; at least one strap with at least one hook attachment device; and at least one attachment device on the at least one panel, wherein the at least one corresponding attachment device on the panel is operative to receive the at least one attachment device on the at least one strap, wherein the at least one corresponding attachment device is positioned on the nursing and pumping bra to provide support for a portion of a breast pump body held adjacent to at least one of a wearer's breast during milk expression performed by the breast pump body.
Clause 15. The nursing and pumping bra of clause 14, wherein the at least one strap may fit around a wearer's neck.
Clause 16. The nursing and pumping bra of clause 14, wherein the at least one strap may be attached to corresponding attachment devices at a center and a left side of the nursing and pumping bra to support the breast pump body on the left side of the nursing and pumping bra.
Clause 17. The nursing and pumping bra of clause 14, wherein the at least one strap is a single loop with a hook attachment device attached to a loop corresponding attachment devices at a center of the nursing and pumping bra.
Clause 18. The nursing and pumping bra of clause 14, wherein the at least one strap is a single loop with one hook attachment device attached to one loop corresponding attachment devices at a center of the nursing and pumping bra.
Clause 19. The nursing and pumping bra of clause 14, wherein the at least one strap is a loop that fits around a wearer's neck and the at least one strap has a first hook attachment device attached to a first loop corresponding attachment devices and a second hook attachment device attached to a second loop corresponding attachment device on the nursing and pumping bra.
   Nonetheless, for the avoidance of doubt, please note that the scope of the invention is to be determined by the claims.

## Claims

1. A nursing and pumping bra, comprising:
an inner pumping panel comprising at least one covering for a wearer's breast, the at least one covering comprising a first layer of fabric that overlaps a second layer of fabric to cover an opening, the at least one covering having stitching extending across a portion of the first layer of fabric and a portion of the second layer of fabric, and the at least one covering is operative to provide support during milk expression for at least one of the wearer's breasts and a portion of a breast pump body inserted through the opening; and
an exterior front surface comprising a first panel of fabric that overlaps at least a portion of a second panel of fabric, the first and the second panels operative to be moved to expose the at least one covering of the inner pumping panel.

2. The nursing and pumping bra of claim 1, wherein the breast pump body sits within the opening and is supported by the first and the second layers of fabric.

3. The nursing and pumping bra of claim 1, wherein the first and the second panels provide support for the breast pump body, when the inner pumping panel is not covered.

4. The nursing and pumping bra of claim 1, wherein the inner pumping panel comprises two coverings, wherein a second covering comprises a third layer of fabric and a fourth layer of fabric to cover an opening, the second covering having stitching extending across a portion of the third layer of fabric and a portion of the fourth layer of fabric.

5. The nursing and pumping bra of claim 1, further comprising:
at least one shoulder strap, a fabric stay, and a strap selectively attached to the nursing and pumping bra to fit around the wearer's neck and provide support for the portion of the breast pump body.

6. The nursing and pumping bra of claim 1, wherein the portion of the breast pump body is a breast shield.

7. The nursing and pumping bra of claim 1, wherein the first panel crosses over the second panel in a closed state to at least partially cover the inner pumping panel.

8. The nursing and pumping bra of claim 1, further comprising:
at least one loop of a fabric attached to the inner pumping panel, the at least one loop operative to support the at least the portion of the breast pump body sitting within the opening of the pumping panel.

9. The nursing and pumping bra of claim 8, wherein the at least one loop is an elastic loop sewn into the inner pumping panel and the at least one loop is operative to fit around a breast shield and secure the breast shield in place within the opening.

10. The nursing and pumping bra of claim 8, wherein the at least one loop is an elastic loop sewn into a shoulder strap on the inner pumping panel.

11. The nursing and pumping bra of claim 1, further comprising:
at least one strap with at least one hook attachment device; and
at least one attachment device on the inner pumping panel, wherein the at least one corresponding attachment device on the inner pumping panel is operative to receive the at least one attachment device on the at least one strap, wherein the at least one corresponding attachment device is positioned on the nursing and pumping bra to provide support for the portion of the breast pump body held adjacent to at least one of the wearer's breasts during milk expression performed by the breast pump body.

12. The nursing and pumping bra of claim 11, wherein the at least one strap may fit around a wearer's neck.

13. The nursing and pumping bra of claim 11, wherein the at least one strap may be attached to corresponding attachment devices at a center and a left side of the nursing and pumping bra to support the breast pump body on the left side of the nursing and pumping bra.

14. The nursing and pumping bra of claim 11, wherein the at least one strap is a single loop with a hook attachment device attached to a loop corresponding attachment devices at a center of the nursing and pumping bra.

15. The nursing and pumping bra of claim 11, wherein the at least one strap is a loop that fits around a wearer's neck and the at least one strap has a first hook attachment device attached to a first loop corresponding attachment devices and a second hook attachment device attached to a second loop corresponding attachment device on the nursing and pumping bra.
